# EUROPEAN PATENT APPLICATION

(11) **EP 4 345 158 A1**
(43) Date of publication of application: **03.04.2024**
(21) Application number: 22837642.2
(22) Date of filing: 04.07.2022
(51) Int. Cl.: C12N 5/00, C12M 1/00, C12N 1/04

(54) **SOLUTION FOR CELL MANIPULATION, CELL CRYOPRESERVATION METHOD, AND CELL THAWING METHOD**

(30) Priority: 05.07.2021 JP 2021111428
(71) Applicant: Advanced Institute of Reproductive Technologies, Tokyo 160-0022 (JP)
(72) Inventor: KUWAYAMA, Masashige, Tokyo 160-0022 (JP); KUWAYAMA, Rio, Tokyo 160-0022 (JP)
(74) Representative: AWA Sweden AB
(86) International application number: PCT/JP2022/026582
(87) International publication number: WO 2023/282227

(57) **Abstract**

Provided are a cell manipulation solution which improves manipulability of a cell, and a cell cryopreservation method and a cell thawing method using this cell manipulation solution. This cell manipulation solution is for use in vitrification of a cell or thawing of a cell subjected to vitrification and comprises a thickener, wherein the thickener contains at least one selected from xanthan gum, carrageenan, gellan gum, locust bean gum, guar gum, and diutan gum. The manipulability of a cell in vitrification of a cell or thawing of a cell subjected to vitrification is improved by using this cell manipulation solution.

## Description

### Technical Field

The present invention relates to a cell manipulation solution for cryopreserving a cell, and a cell cryopreservation method and a cell thawing method using this cell manipulation solution.

### Background Art

Recently, in fertility treatment of humans, cryopreservation of eggs and embryos has been attracting attention.

Conventionally, as methods for cryopreserving eggs or the like of mammals, a method including: enclosing and bonding eggs, embryos, or the like to a vial or a straw with a vitrification solution; sealing a cryopreservation container; and then rapidly cooling the cryopreservation container by bringing the cryopreservation container into contact with liquid nitrogen has been known (for example, Patent Literature 1).

In addition, a vitrification preservation technique that enables preservation of fertilized eggs while hardly damaging the fertilized eggs has been reported by Kuwayama et. al.

A preservation solution for use in vitrifying and preserving fertilized eggs has been reported (for example, Patent Literature 2). This preservation solution does not contain serum and contains at least one water-soluble cellulose-based cryoprotectant selected from the group consisting of hydroxypropyl cellulose, hydroxypropyl methyl cellulose, and hydroxyethyl methyl cellulose as a cryoprotectant.

### Citation List

### Patent Literatures

Patent Literature 1: Japanese Unexamined Patent Publication No. 2000-189155
Patent Literature 2: Japanese Unexamined Patent Publication No. 2011-111406

### Summary of Invention

### Technical Problem

Preservation solutions for vitrifying cells (fertilized eggs) are required to have properties such as easiness in manipulation cells in the case of cryopreservation manipulation in addition to safety. Then, cryopreservation manipulation is required to be capable of shortening working hours and easy to conduct in consideration of loads on cells.

In view of this, an object of the present invention is to provide a cell manipulation solution that improves manipulability of cells, a cell cryopreservation method and a cell thawing method using this cell manipulation solution.

### Solution to Problem

A cell manipulation solution according to the present invention is for use in vitrification of a cell or thawing of a cell subjected to vitrification and comprises a thickener, wherein the thickener is at least one selected from xanthan gum, carrageenan, gellan gum, locust bean gum, guar gum, and diutan gum.

In the cell manipulation solution according to the present invention, the thickener has a concentration of from 0.01% by mass to 0.50% by mass and a viscosity of from 1.5 mPa·s to 100 mPa·s.

The cell manipulation solution according to the present invention comprises:
at least one selected from cell membrane-permeable cryoprotectants of dimethyl sulfoxide, ethylene glycol, glycerol, propylene glycol, and butanediol; and
at least one selected from cell membrane-impermeable cryoprotectants of trehalose, polyvinylpyrrolidone, sucrose, and polyethylene glycol.

A cell cryopreservation method according to the present invention uses a plate including at least a first well and a second well which are filled with the above-described cell manipulation solution, wherein a viscosity of a thickener in a first cell manipulation solution with which the first well is filled is lower than a viscosity of a thickener in a second cell manipulation solution with which the second well is filled, the cell cryopreservation method comprising: a first step of immersing the cell in the first cell manipulation solution in the first well; a second step of, after the first step, taking out the cell from the first cell manipulation solution in the first well and immersing the cell in the second cell manipulation solution in the second well; and a third step of, after the second step, taking out the cell from the second cell manipulation solution in the second well and freezing the cell.

A cell thawing method according to the present invention is a cell thawing method for thawing a cell by using a plate including a first well, a second well, a third well, and a fourth well which are filled with the above-described cell manipulation solution, wherein a viscosity of a thickener in a fourth cell manipulation solution with which the fourth well is filled is higher than viscosities of thickeners in a first cell manipulation solution with which the first well is filled, a second cell manipulation solution with which the second well is filled, and a third cell manipulation solution with which the third well is filled, the cell thawing method comprising: a first step of immersing the cell in the fourth cell manipulation solution in the fourth well to thaw the cell; a second step of, after the first step, taking out the cell from the fourth cell manipulation solution in the fourth well and immersing the cell in the first cell manipulation solution in the first well; and a third step of, after the second step, taking out the cell from the first cell manipulation solution in the first well and immersing the cell in the second cell manipulation solution in the second well.

### Advantageous Effects of Invention

The cell manipulation solution of the present invention contains a thickener. This thickener contains at least one of xanthan gum, carrageenan, gellan gum, locust bean gum, guar gum, and diutan gum. Since the cell manipulation solution contains these thickeners, a cell slowly sediments or floats in the cell manipulation solution when the cell is vitrified, for example. This makes it possible to improve the manipulability, reduce the work burden, and shorten the manipulation time.

The cell cryopreservation method of the present invention uses the above-mentioned cell manipulation solution as an equilibration solution and a vitrification solution, and as the equilibration solution, a cell manipulation solution having a lower viscosity of the thickener in the vitrification solution is used. Then, after the cell is immersed in the equilibration solution, the cell is immersed in the vitrification solution to be frozen. This cell cryopreservation method improves the manipulability of a cell and can vitrify a cell in a shorter period of time than the conventional techniques.

The cell thawing method of the present invention uses the cell manipulation solution as a thawing solution, a dilution solution, and a washing solution, and a viscosity of the thickener in the thawing solution is made higher than viscosities of the dilution solution and the washing solution. Then, after the cell is immersed in the thawing solution, the cell is immersed in the dilution solution, and the cell is further immersed in the washing solution. This cell thawing method improves the manipulability of a cell and can thaw and use a cell in a shorter period of time than the conventional techniques.

### Brief Description of Drawings

[Fig. 1] Fig. 1 is a perspective view of a cell freezing device filled with a cell manipulation solution according to a first embodiment of the present invention.
[Fig. 2] Fig. 2 is a sectional view of the cell freezing device filled with the cell manipulation solution according to the first embodiment of the present invention.
[Fig. 3] Fig. 3 is a perspective view of a cell thawing device filled with a cell manipulation solution according to a second embodiment of the present invention.
[Fig. 4] Fig. 4 is a sectional view of the cell thawing device filled with the cell manipulation solution according to the second embodiment of the present invention.

### Description of Embodiments

### [First Embodiment]

A cell manipulation solution according to the present embodiment is used as a cell vitrification solution.

This cell vitrification solution is used for cryopreserving sperms, eggs, or embryos of mammals, for example. This cell vitrification solution contains a thickener, and contains, for example, at least one of xanthan gum, carrageenan, gellan gum, locust bean gum, guar gum, and diutan gum, which are thickening polysaccharides.

Note that the cell vitrification solution does not contain hydroxypropyl cellulose, hydroxypropyl methyl cellulose, or hydroxyethyl methyl cellulose.

The thickener is preferably contained at a concentration of from 0.01% by mass to 0.50% by mass relative to the cell vitrification solution. In addition, the concentration is most preferably from 0.03% by mass to 0.30% by mass. In the case where the concentration of the thickener is lower than 0.01% by mass, there is a possibility that the effect of an increase in viscosity is lost, and cells come into contact with each other to be bonded in the cell vitrification solution, so that the manipulability in manipulation one cell decreases. On the other hand, in the case where the concentration of the thickener is higher than 0.50% by mass, the viscosity of the cell vitrification solution increases, so that the manipulability of cells in the cell vitrification solution decreases.

In addition, the viscosity of the cell vitrification solution is preferably from 1.5 mPa·s to 100 mPa·s. In the case where the viscosity of the cell vitrification solution is smaller than 1.5 mPa·s, there is a possibility that cells come into contact with each other to be bonded in the cell vitrification solution, so that the manipulability in manipulation one cell decreases. On the other hand, in the case where the viscosity of the cell vitrification solution is larger than 100 mPa·s, it becomes difficult to take out cells in the cell vitrification solution, so that the manipulability of cells decreases.

Note that the viscosity of each solution was measured by using a tuning fork vibration viscometer (22°±1.0°).

The cell vitrification solution contains a plurality of salts, a plurality of amino acids, plurality of buffering agents, and the like. Moreover, this cell vitrification solution contains at least one selected from cell membrane-permeable cryoprotectants of dimethyl sulfoxide, ethylene glycol, glycerol, propylene glycol, and butanediol and contains at least one selected from cell membrane-impermeable cryoprotectants of trehalose, polyvinylpyrrolidone, sucrose, and polyethylene glycol.

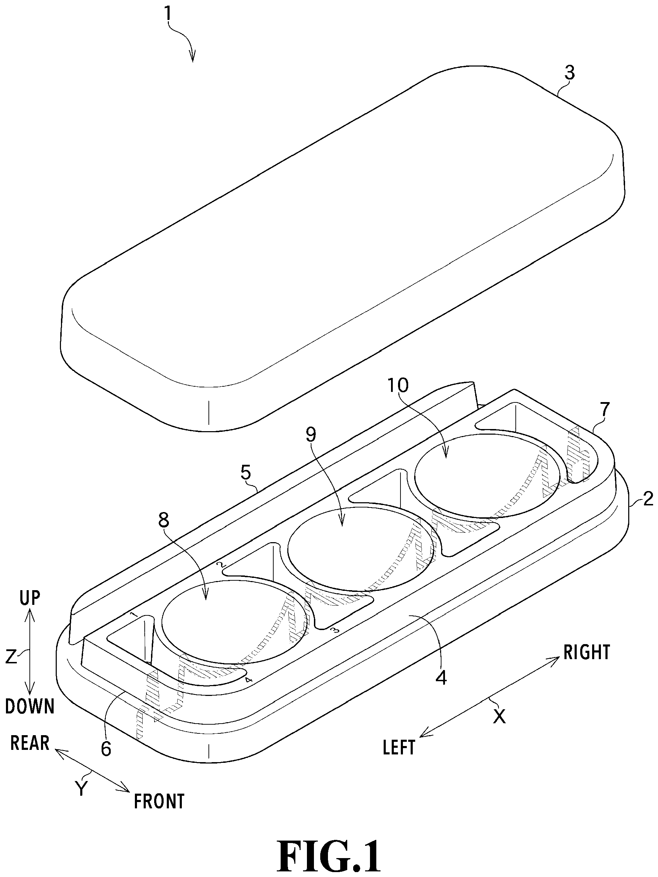

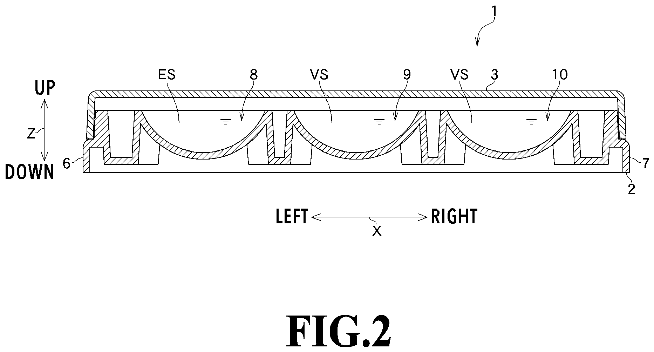

Next, a cell freezing device 1 to be filled with the cell vitrification solution will be described with reference to Fig. 1 and Fig. 2. This cell freezing device 1 can be used in freezing various cells. In the present embodiment, the case of using the cell freezing device 1 in freezing fertilized eggs (blastocyst) will be described as an example.

As shown in Fig. 1, the cell freezing device 1 according to the present embodiment includes: a plate body 2; and a lid member 3 which covers this plate body 2 from above. Hereinafter, based on Fig. 1, a direction of the longer sides of the plate body 2 is referred to as a right-left direction X, a direction of the shorter sides of the plate body 2 is referred to as a front-rear direction Y, and a direction of the height of the plate body 2 is referred to as an up-down direction Z.

The plate body 2 has a laterally long shape, and has a front wall 4, a rear wall 5 opposite to the front wall 4, and a left wall 6 and a right wall 7 formed continuously to the front wall 4 and the rear wall 5. This plate body 2 is formed of a transparent material, and is formed of, for example, polypropylene, polystyrene, acrylic resin, or glass. The size of the plate body 2 is designed such that the length L1 in the right-left direction X is from 5.5 cm to 7.5 cm, and the length L2 in the front-rear direction Y is from 2 cm to 3.5 cm.

As shown in Fig. 2, the plate body 2 has substantially semi-spherical first well 8, second well 9, and third well 10 formed in the right-left direction X. The diameters and depths of these first well 8 to third well 10 are designed substantially the same. In the first well 8, an equilibration solution ES (Equilibration Solution) is injected, and the second well 9 and the third well 10 are each filled with a vitrification solution VS (VS: Vitrification Solution). The cell vitrification solution described above is used as the equilibration solution ES and the vitrification solution VS.

In the present embodiment, the vitrification solution VS is a solution containing a cryoprotectant necessary to vitrify cells, and this vitrification solution VS contains a basal medium (MEM), an antibiotic, and the like. On the other hand, the equilibration solution ES is a solution containing a cryoprotectant at a lower concentration than that of the vitrification solution VS, and this equilibration solution ES contains a basal medium (MEM) and an antibiotic.

Next, a cell cryopreservation method (cell vitrification method) using the cell vitrification solution will be described.

In the cell cryopreservation method according to the present embodiment, cell vitrification solutions having different concentrations and viscosities of a thickener (thickening polysaccharide) are used as the equilibration solution ES and the vitrification solution VS. The concentration of the thickener in the equilibration solution ES is preferably lower than the concentration of the thickener in the vitrification solution VS. Moreover, the viscosity of the thickener in the equilibration solution ES is preferably lower than the viscosity of the thickener in the vitrification solution VS. In the present embodiment, the concentration of the thickener in the equilibration solution ES is set to 0.048% by mass, and the concentration of the thickener in the vitrification solution VS is set to 0.060% by mass. In addition, it is preferable that the viscosity of the thickener in the equilibration solution ES be from 2.0 to 4.0 mPa·s, and the viscosity of the thickener in the vitrification solution VS be from 7.0 to 13 mPa·s.

The equilibration solution ES (first cell manipulation solution), the vitrification solution VS (second cell manipulation solution), and the vitrification solution VS (third cell manipulation solution) are dispensed each in an amount of 300 µL into the first well 8, the second well 9, and the third well 10, respectively. After the equilibration solution ES and the vitrification solutions VS are dispensed, a plurality of fertilized eggs are put into the first well 8. Since the equilibration solution ES has a predetermined viscosity, the fertilized eggs thus put in slowly sediment to the bottom. By causing the fertilized eggs to slowly sediment to the bottom, the entire surfaces of the fertilized eggs are immersed into the equilibration solution ES, and the cryoprotectant permeates the insides of the fertilized eggs, and the fertilized eggs are equilibrated. In addition, since the equilibration solution ES has a predetermined viscosity, the fertilized eggs are unlikely to come into contact with each other in the equilibration solution ES in the first well 8, making it easy to manipulate each cell.

Next, after it is confirmed that the fertilized eggs are turned into a desired form, the fertilized egg and the equilibration solution ES are sucked in from inside the first well 8 by using a capillary, and the fertilized egg and the equilibration solution ES thus sucked in are put into the bottom of the second well 9. Since the vitrification solution VS has a predetermined viscosity, the fertilized egg put in slowly floats up to near the surface of the vitrification solution VS. The capillary is washed with the vitrification solution VS inside the second well 9 and the third well 10, and the fertilized egg having floated and the vitrification solution VS inside the second well 9 are sucked in. The fertilized egg and the vitrification solution VS thus sucked in are put into the third well 10.

Since the vitrification solution VS has a predetermined viscosity, the fertilized egg put in slowly floats up in the vitrification solution VS. The vitrification solution VS in the third well 10 is stirred with the capillary, the state of the fertilized egg having floated is checked, and thereafter, the capillary is washed with the vitrification solution VS inside the third well 10. Then, the fertilized egg sucked in with the capillary is dropped onto an end of a bar-shaped cell freezing device and frozen with liquid nitrogen.

The above manipulation is conducted on each fertilized egg which has sedimented in the first well 8 to freeze the plurality of fertilized eggs.

Next, the operations and effects of the cell vitrification solution and the cell cryopreservation method according to the present embodiment will be described.

Since the cell vitrification solution according to the present embodiment contains a thickener and has a predetermined viscosity, the sedimentation rate and the floating rate of cells in the cell vitrification solution can be adjusted, making it easy to manipulate the cells while ensuring the safety. Hence, it is possible to reduce work burden and shorten manipulation time. In particular, in the case where the concentration of the thickener in the cell vitrification solution is from 0.01% by mass to 0.50% by mass, and in the case where the viscosity of the cell vitrification solution is from 1.5 mPa·s to 100 mPa·s, it is possible to obtain good manipulability in manipulation a plurality of cells, and achieve manipulation in a short period of time.

Fertilized eggs slowly sediment in the equilibration solution ES. As compared with a case where fertilized eggs rapidly sediment in a solution, the time during which the entire fertilized eggs (particularly, their lower parts) are in contact with the cell vitrification solution is longer, making it possible to more stably vitrify the fertilized eggs. Then, since fertilized eggs slowly come into contact with the bottom of the container in the equilibration solution ES, the load on the fertilized eggs is reduced. In addition, fertilized eggs slowly float in the vitrification solution VS in the second well 9, it is possible to prevent fertilized eggs from being visually lost in the vitrification solution VS.

The states of fertilized eggs turning into a desired form in a conventional vitrification cryopreserving solution containing a cellulose-based substance and the cell vitrification solution according to the present embodiment were observed to inspect the permeability of a cell-permeable cryoprotectant into the fertilized eggs. As a result, it was found that the cell-permeable cryoprotectant more easily permeated the fertilized eggs in the case where the cell vitrification solution of the present embodiment was used. Hence, it is possible to rapidly conduct vitrification process on fertilized eggs and reduce the load on the fertilized eggs as compared with the case of using the conventional solution.

Among the above-mentioned thickening polysaccharides, a cell vitrification solution containing only xanthan gum is particularly useful. In addition, cell vitrification solutions containing two or more of xanthan gum, carrageenan, gellan gum, locust bean gum, guar gum, and diutan gum, which are thickening polysaccharides, are also highly effective. Moreover, it was found that in the case where the concentration of the thickener was from 0.03% by mass to 0.30% by mass, the manipulability of cells was significantly good.

In the cell cryopreservation method according to the present embodiment, the cell manipulability is improved by conducting the cell cryopreservation method by using the above-mentioned cell vitrification solution, changing the concentrations and viscosities of the thickener in the equilibration solution ES and the vitrification solution VS to adjust the sedimentation rates and the floating rates of fertilized eggs. Hence, the time taken for fertilized eggs to turn into a desired form is shortened as compared with the conventional cryopreservation methods. Therefore, this cell cryopreservation method enables cells to be more safely frozen, and the cell cryopreservation method according to the present embodiment is highly useful.

### [Second Embodiment]

The cell manipulation solution according to the present embodiment can be also used as a cell thawing solution for cells subjected to vitrification. This cell thawing solution is used for the purpose of improving the manipulability of cell thawing manipulation.

Conventionally, there has been a case where a vitrification cryopreserving solution (for example, a vitrification cryopreserving solution for animal cells described in Patent Literature 2) is used as a thawing solution for thawing cells. However, this solution has a problem that if the solution is warmed to about 37°, a cellulose-based substance precipitates.

The cell thawing solution according to the present embodiment contains at least one of xanthan gum, carrageenan, gellan gum, locust bean gum, guar gum, and diutan gum, which are thickening polysaccharides, and does not contain hydroxypropyl cellulose, hydroxypropyl methyl cellulose, or hydroxyethyl methyl cellulose.

The thickener is preferably contained at a concentration of from 0.01% by mass to 0.50% by mass relative to the cell thawing solution. In addition, it is most preferable that the concentration be from 0.03% by mass to 0.30% by mass. In the case where the concentration of the thickener is lower than 0.01% by mass, there is a possibility that an increase in viscosity is lost and the manipulability decreases. On the other hand, in the case where the concentration of the thickener is higher than 0.50% by mass, the viscosity of the cell thawing solution increases, so that the manipulability of cells in the cell vitrification solution decreases.

In addition, the viscosity of the cell thawing solution is preferably from 1.5 mPa·s to 100 mPa·s. In the case where the viscosity of the cell thawing solution is smaller than 1.5 mPa·s, there is a possibility that an increase in viscosity is lost and the manipulability decreases. On the other hand, in the case where the viscosity of the cell thawing solution is larger than 100 mPa·s, the viscosity of the cell thawing solution increases, so that the manipulability of cells in the cell vitrification solution decreases.

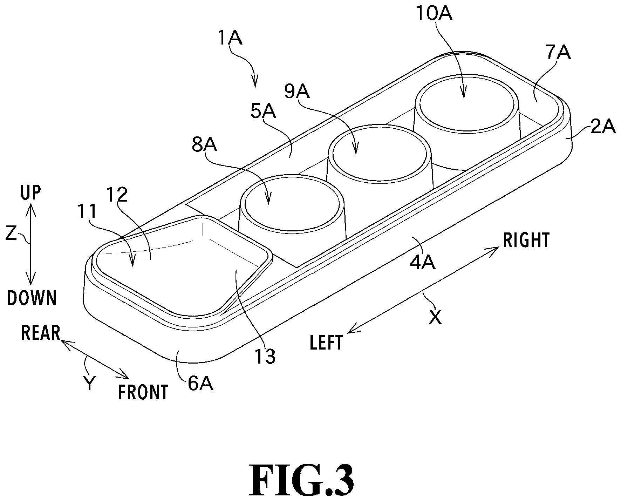

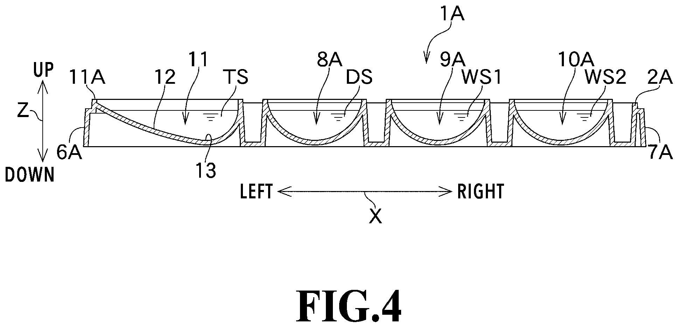

Next, a cell thawing device 1A to be filled with the cell thawing solution will be described with reference to Fig. 3 and Fig. 4. This cell thawing device 1A can be used in thawing various cells. In the present embodiment, the case of using the cell thawing device 1A in thawing frozen fertilized eggs (blastocyst) will be described as an example.

As shown in Fig. 3, the cell thawing device 1A includes a plate body 2A laterally long in the right-left direction X. Note that a sealing material which is attached to an upper surface of the plate body 2A to be capable of being peeled may be provided. The plate body 2A has a front wall 4A, a rear wall 5A opposite to the front wall 4A, and a left wall 6A and a right wall 7A formed continuously to the front wall 4A and the rear wall 5A. For this plate body 2A, a transparent, light-weight material, for example, polypropylene, polystyrene, polycarbonate, or acrylic resin is used.

The plate body 2A has a semi-spherical first well 8A, a semi-spherical second well 9A, a semi-spherical third well 10A, and a fourth well 11 which is substantially trapezoidal in a plan view, formed in the right-left direction X. The first well 8A, the second well 9A, and the third well 10A are formed to protrude from a bottom wall of a groove portion of the plate body 2A. The first well 8A is formed on the right side from the fourth well 11 in the right-left direction X, the second well 9A is formed on the right side from the first well 8A, and the third well 10A is formed on the right side from the second well 9A, respectively.

As shown in Fig. 4, the fourth well 11 includes a bottom wall 13 having an inclination portion 12 which inclines from the left to the right in the right-left direction X.

The fourth well 11 is filled with a thawing solution TS (Thawing Solution). On the other hand, the first well 8A is filled with a dilution solution DS (Dilution Solution), the second well 9A is filled with a washing solution WS 1 (Washing Solution 1), and the third well 10A is filled with a washing solution WS2 (Washing Solution 2).

The cell thawing solution according to the present embodiment is used as the thawing solution TS, the dilution solution DS, and the washing solutions WS1, WS2.

In the present embodiment, the thawing solution TS and the dilution solution DS contain a basal medium (MEM), an antibiotic, a cell-impermeable dehydration promoting substance (for example, trehalose), and the like. The washing solutions WS1, WS2 contain a basal medium (MEM) and an antibiotic and do not contain a cell-impermeable dehydration promoting substance (for example, trehalose).

Next, a cell thawing method using the cell thawing solution will be described.

In the cell thawing method according to the present embodiment, cell thawing solutions having different concentrations and viscosities of a thickener are used as the thawing solution TS, the dilution solution DS, and the washing solutions WS1, WS2. The concentration and the viscosity of the thickener in the thawing solution TS are preferably higher than the concentrations and the viscosities of the thickeners in the dilution solution DS and the washing solutions WS1, WS2. In the present embodiment, the concentration of the thickener in the thawing solution TS is set to 0.06% by mass, the concentration of the thickener in the dilution solution DS is set to 0.06% by mass, and the concentration of the thickener in the washing solutions WS1, WS2 is set to 0.03% by mass. It is preferable that the viscosity of the thickener in the thawing solution TS be from 7.0 to 15 mPa·s, the viscosity of the thickener in the dilution solution DS be from 2.5 to 4.0 mPa·s, and the viscosity of the thickener in the washing solutions WS1, WS2 be from 1.5 to 2.0 mPa·s.

Next, the cell thawing method using the above-mentioned cell thawing solution will be described.

The fourth well 11 is filled with the thawing solution TS (fourth cell thawing solution), the first well 8A is filled with the dilution solution DS (first cell thawing solution), the second well 9A is filled with the washing solution WS1 (second cell thawing solution), and the third well 10A is filled with the washing solution WS2 (third cell thawing solution). Then, fertilized eggs frozen on a cell manipulation device are immersed into the thawing solution TS. Since the thawing solution TS has a predetermined viscosity, the thawing solution TS slowly permeates the frozen fertilized eggs. Thereafter, the fertilized eggs thus thawed separate from the cell manipulation device and slowly float in the thawing solution TS.

Then, the floating fertilized eggs are put into the dilution solution DS in the first well 8A and diluted for 3 minutes. Next, the fertilized eggs thus diluted are put into the washing solution WS1 in the second well 9A and washed for 5 minutes. At this time, it is checked that the fertilized eggs are in a desired form. After the form is checked, the fertilized eggs are put into the washing solution WS2 in the third well 10A to complete washing. After the washing is completed, the fertilized eggs are used for embryo transfer and the like.

Next, operations and effects of the cell thawing solution and the cell thawing method according to the present embodiment will be described.

The cell thawing solution according to the present embodiment contains a thickener, and the floating rate of cells in the cell thawing solution can be adjusted, making it easy to conduct cell thawing manipulation while ensuring safety. In particular, in the case where the concentration of the thickener in the cell thawing solution is from 0.01% by mass to 0.50% by mass, and in the case where the viscosity of the cell thawing solution is from 1.5 mPa·s to 100 mPa·s, it is possible to obtain good manipulability in manipulation a plurality of cells, and achieve manipulation in a short period of time. Among the above-mentioned thickening polysaccharides, a cell thawing solution containing xanthan gum is particularly useful.

In addition, even in the case where the cell thawing solution according to the present embodiment is warmed to 37°, no substance precipitates, and cells can be thawed safely.

In a conventional thawing solution, since the thawing solution easily permeates fertilized eggs, there has been a possibility that after fertilized eggs are immersed in the thawing solution, the fertilized eggs immediately separate from a cell manipulation device, which result in loosing track of the fertilized eggs.

Since the thawing solution TS of the present embodiment has a predetermined viscosity, the thawing solution TS gradually permeates fertilized eggs immersed in the thawing solution TS, and after separating from the cell manipulation device, the fertilized eggs slowly float in the thawing solution TS. Hence, it is possible to prevent loosing track of the fertilized eggs in the thawing solution TS.

The cell thawing method according to the present embodiment is conducted by using the above-mentioned cell thawing solution. The manipulability of fertilized eggs in the thawing solution TS and the like is improved by changing the concentrations and viscosities of the thickeners in the thawing solution TS, the dilution solution DS, and the washing solutions WS1, WS2, and the time required for fertilized eggs to turn into a desired form is shortened as compared with the conventional thawing methods. In particular, the manipulability of cells is enhanced by making the viscosity of the thickener in the thawing solution TS higher than the viscosities of the thickeners in the dilution solution DS and the washing solutions WS1, WS2. Therefore, this cell thawing method enables cells to be more safely manipulated, and the cell thawing method according to the present embodiment is highly useful.

The present embodiments have been described above; however, besides these, it is possible to select the configurations given in the above-described embodiments or change the configurations to other configurations as appropriate without departing from the gist of the present invention.

The polysaccharide is not limited to xanthan gum, carrageenan, gellan gum, locust bean gum, guar gum, and diutan gum as long as the polysaccharide has high safety and thickening effect, and, for example, hyaluronic acid, rhamsan gum, hexuronic acid, fucoidan, pectin, and the like can also be used.

In addition, the cell manipulation solution can be used in manipulations other than freezing or thawing of cells as long as the cell manipulation solution is used as a solution for manipulating cells.

### Reference Signs List

- 1: cell freezing device
- 1A: cell thawing device
- 2, 2A: plate body (main body part)
- 3: lid member
- 4, 4A: front wall
- 5, 5A: rear wall
- 6, 6A: left wall
- 7, 7A: right wall
- 8, 8A: first well
- 9, 9A: second well
- 10, 10A: third well
- 11: fourth well
- 12: inclination portion
- 13: bottom wall
- ES: equilibration solution (first cell manipulation solution)
- VS: vitrification solution (second cell manipulation solution, third cell manipulation solution)
- TS: thawing solution (fourth cell manipulation solution)
- DS: dilution solution (first cell manipulation solution)
- WS1, WS2: washing solution (second cell manipulation solution, third cell manipulation solution)
- X: right-left direction
- Y: front-rear direction
- Z: up-down direction

## Claims

1. A cell manipulation solution for use in vitrification of a cell or thawing of a cell subjected to vitrification, comprising:
a thickener, wherein
the thickener is at least one selected from xanthan gum, carrageenan, gellan gum, locust bean gum, guar gum, and diutan gum.

2. The cell manipulation solution according to claim 1, wherein
the thickener has a concentration of from 0.01% by mass to 0.50% by mass and a viscosity of from 1.5 mPa·s to 100 mPa·s.

3. The cell manipulation solution according to claim 1 or 2, comprising:
at least one selected from cell membrane-permeable cryoprotectants of dimethyl sulfoxide, ethylene glycol, glycerol, propylene glycol, and butanediol; and
at least one selected from cell membrane-impermeable cryoprotectants of trehalose, polyvinylpyrrolidone, sucrose, and polyethylene glycol.

4. A cell cryopreservation method for vitrification of a cell by using a plate including at least a first well and a second well which are filled with the cell manipulation solution according to claim 1, wherein
a viscosity of a thickener in a first cell manipulation solution with which the first well is filled is lower than a viscosity of a thickener in a second cell manipulation solution with which the second well is filled,
the cell cryopreservation method comprising:
a first step of immersing the cell in the first cell manipulation solution in the first well;
a second step of, after the first step, taking out the cell from the first cell manipulation solution in the first well and immersing the cell in the second cell manipulation solution in the second well; and
a third step of, after the second step, taking out the cell from the second cell manipulation solution in the second well and freezing the cell.

5. A cell thawing method for thawing a cell by using a plate including a first well, a second well, a third well, and a fourth well which are filled with the cell manipulation solution according to claim 1, wherein
a viscosity of a thickener in a fourth cell manipulation solution with which the fourth well is filled is higher than viscosities of thickeners in a first cell manipulation solution with which the first well is filled, a second cell manipulation solution with which the second well is filled, and a third cell manipulation solution with which the third well is filled,
the cell thawing method comprising:
a first step of immersing the cell in the fourth cell manipulation solution in the fourth well to thaw the cell;
a second step of, after the first step, taking out the cell from the fourth cell manipulation solution in the fourth well and immersing the cell in the first cell manipulation solution in the first well; and
a third step of, after the second step, taking out the cell from the first cell manipulation solution in the first well and immersing the cell in the second cell manipulation solution in the second well.
